(19) Europäisches Patentamt
European Patent Office
Office européen des brevets

(11) **EP 2 903 937 B1**

(12) **EUROPEAN PATENT SPECIFICATION**

(45) Date of publication and mention
of the grant of the patent:
**27.07.2016 Bulletin 2016/30**

(21) Application number: **13801777.7**

(22) Date of filing: **24.09.2013**

(51) Int Cl.:
*C01F 7/02* (2006.01)   *C04B 26/06* (2006.01)
*C04B 38/00* (2006.01)   *C25D 11/04* (2006.01)
*C25D 11/18* (2006.01)   *A61K 6/083* (2006.01)
*C04B 111/00* (2006.01)   *A61K 6/00* (2006.01)

(86) International application number:
**PCT/IB2013/058809**

(87) International publication number:
**WO 2014/053946 (10.04.2014 Gazette 2014/15)**

(54) **COMBINED MATERIAL INCLUDING ANODIC POROUS ALUMINA AND A POLYMER MATRIX, AND ITS USE FOR THE DENTAL RECONDITION**

KOMBINIERTES MATERIAL MIT ANODISCHEM PORÖSEM ALUMINIUMOXID UND EINER POLYMERMATRIX UND DESSEN VERWENDUNG FÜR ZAHNÄRZTLICHE WIEDERHERSTELLUNGSEINGRIFFE

MATÉRIAU COMBINÉ COMPRENANT DE L'ALUMINE POREUSE ANODIQUE ET UNE MATRICE POLYMÈRE, ET SON UTILISATION POUR LE RECONDITIONNEMENT DENTAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.10.2012 IT MI20121634**

(43) Date of publication of application:
**12.08.2015 Bulletin 2015/33**

(73) Proprietor: **Fondazione Istituto Italiano Di Tecnologia**
**16163 Genova (IT)**

(72) Inventors:
• **SALERNO, Marco**
  **I-16149 Genova (IT)**
• **THORAT, Sanjay**
  **I-16162 Genova (IT)**
• **DIASPRO, Alberto**
  **I-16124 Genova (IT)**

(74) Representative: **Biggi, Cristina**
**c/o Bugnion S.p.A.**
**Viale Lancetti 17**
**20158 Milano (IT)**

(56) References cited:
**US-A1- 2002 193 463    US-A1- 2011 203 928**

• **CHRISTOPHE AZEVEDO ET AL: "Structure and surface reactivity of novel nanoporous alumina fillers", JOURNAL OF BIOMEDICAL MATERIALS RESEARCH PART B: APPLIED BIOMATERIALS, vol. 88B, no. 1, 2009, pages 174-181, XP055071519, ISSN: 1552-4973, DOI: 10.1002/jbm.b.31165**
• **Marco Salerno ET AL: "Biomaterials for the programming of cell growth in oral tissues : The possible role of APA", Bioinformation, volume 5, issue 7, 6 January 2011 (2011-01-06), pages 291-293, XP055072046, Retrieved from the Internet: URL:http://www.ncbi.nlm.nih.gov/pmc/articl es/PMC3043349/pdf/97320630005291.pdf [retrieved on 2013-07-19] cited in the application**
• **LI A P ET AL: "Polycrystalline nanopore arrays with hexagonal ordering on aluminum", JOURNAL OF VACUUM SCIENCE AND TECHNOLOGY: PART A, AVS /AIP, MELVILLE, NY., US, vol. 17, no. 4, July 1999 (1999-07), pages 1428-1431, XP012004557, ISSN: 0734-2101, DOI: 10.1116/1.581832 cited in the application**
• **NURSEL DILSIZ ET AL: "Study of sol-gel processing for fabrication of low density alumina microspheres", MATERIALS SCIENCE AND ENGINEERING A, vol. 332, no. 1-2, July 2002 (2002-07), pages 91-96, XP055072104, ISSN: 0921-5093, DOI: 10.1016/S0921-5093(01)01726-9**

**EP 2 903 937 B1**

## Description

Field of the invention

The present invention relates in general terms to anodic porous alumina (APA) having interconnected through pores, in the form of microparticles, and its use in the preparation of a composite material that is useful in the field of conservative dentistry, in particular as a filling material for dental restoration.

Prior art

In the field of conservative dentistry, ample use is made of restorative dental materials, typically in cases in which cavities need to be filled due to caries removal or fractures. Said restorative dental materials must be able to bond stably to the dental surface, reproduce the behaviours of the original tissues as closely as possible, and be stable and resistant over time.

Up to now, dental restoration operations have provided for the use of composite resins, mainly silica-based, which have nearly entirely replaced the traditional amalgams containing mercury. Although the latter have a high mechanical performance (modulus of elasticity, high degree of hardness and resistance to deterioration), their suspected and probable toxicity has greatly limited their use.

The category of composite materials most used is that of so-called "hybrid" composite materials, which include a combination of inorganic fillers (consisting essentially of glass), covered by a silane coupling agent which joins them to a polymer matrix, and having a broad size distribution, from 10 nm to 10 $\mu$m. However, current restorative composite materials show a stability over time which is considerably inferior compared to amalgams, due in part to the chemical degradation of the filling material, and in part to hydrolysis of the silane coupling agent. The degradation of the coupling agent, associated with the infiltrations that occur in the organic matrix, which is permeable to liquids coming from the surrounding environment (oral cavity), causes, moreover, the release of polymer matrix and coupling material residues in the mouth, which in the long term can be a risk factor for the patient's health.

The possible applications of restructuring composite materials comprising APA in the biological, medical and dental fields are also known in the art.

APA is a nanostructured, inert, biocompatible, highly resistant nontoxic material which can be easily prepared via controlled anodization of an electrode made of super-pure aluminium (i.e. with a purity of 99.999%), according to methods known in the art and described for example in A.P. Li et al. Journal of Vacuum Science & Technology A 17:1428 (1999).

M. Salerno et al. (Bioinformation 5(7), 291-293, 2011) describe a nanocomposite material consisting of APA and an organic polymer, in which the anodic porous alumina is present as a whole material, not broken down into particles.

S. Praveen et al. (Dental Materials 25, 296-301, 2009) describe composite dental materials consisting of a resin matrix and mesoporous silica particles, obtained through a sintering process.

EP0803241 (GC Dental Product Corporation) discloses the use of silane agents as the coupling agents necessary for the adhesion of the polymer matrix to the inorganic particles of the filler.

US2011203928 A1 discloses a mesoporous alumina having a particle size ranging from 1 to 10 $\mu$m, and pores of diameter ranging from 2 to 100 nm.

The applicant has now developed a composite material that is useful in dental restoration and comprises nanoporous alumina having interconnected through pores, in the form of microparticles, and a polymer matrix. Surprisingly, thanks to the mechanical interlace that is established between the microparticles of nanoporous alumina and the polymer matrix, the composite material of the invention does not require the use of any coupling agent, further ensuring excellent properties in terms of resistance, elasticity, biocompatibility and stability over time. In fact, the particular microparticulate form of the nanoporous alumina and the presence of interconnected through holes in each microparticle makes it possible to achieve an almost complete penetration of the polymer matrix into the alumina nanopores. In this manner the two components of the composite material are physically interconnected without there being a need to use any type of chemical coupling agent.

Summary of the invention

In a first aspect, the invention relates to an anodic porous alumina (APA) having interconnected through nanopores and which is in the form of microparticles, optionally functionalized with at least one biologically active agent.

In a further aspect, the invention relates to a process for the formation of microparticles of the above-described nanoporous alumina, which comprises the steps of:

a) preparing a layer of APA by anodic oxidation (anodization) an aluminium, preferably super-pure, electrode, immersed in an electrolytic solution;
b) forming a membrane of APA having interconnected through nanopores, by removing the residual aluminium substrate and subsequently by removing the bottom of the pores of the porous anodic alumina layer, and
c) grinding the alumina membrane of step b), obtaining microparticles of APA having interconnected through nanopores.

In a preferred embodiment of the invention, the process of forming the alumina microparticles of alumina further comprises a step of functionalization of the APA with a biologically active agent, which can be carried out *in situ*, i.e. during the anodization of the aluminium electrode.

In fact, during anodic oxidation, which constitutes the step of growing a layer of APA on the aluminium electrode, it is possible to modify the chemical composition of the APA by appropriately varying the composition of the electrolytic solution and/or the anodization parameters.

As an alternative or in addition to the optional *in-situ* functionalization during the anodization step a), the process of the invention can further comprise an *ex-situ* step of functionalizing the APA, i.e. performed after the formation of the APA, preferably between step b) and step c).

The nanoporous APA in the form of microparticles obtained with the process of the invention can be mixed with a conventional polymer matrix, without the addition of any chemical coupling agent, in order to prepare a composite material that is useful for dental restoration.

Therefore, the present invention also relates to a composite material useful for dental restoration, comprising at least:

- a polymer matrix, and
- an anodic porous alumina having interconnected through nanopores, and which is in the form of microparticles.

In a further aspect, the invention relates to a process for preparing said composite material, comprising the preparation of the microparticulate nanoporous anodic alumina as described above, followed by at least a step of mixing the microparticles of alumina with a polymer matrix.

In a further aspect, the invention relates to the composite material for use as a medicament, preferably in the field of the conservative dentistry, more preferably for dental restoration, in particular as a filler for tooth parts that are missing due, for example, to caries removal or fractures or breaks.

In a further aspect, the invention relates to a use of the present composite material as a cosmetic agent in the field of the conservative dentistry, preferably for dental restoration.

Moreover, an additional aspect is a method for dental restoration, preferably cosmetic, which comprises applying the composite material of the invention in the dental part to be restored and subsequently photopolymerizing the composite material applied.

In a further aspect, the invention also relates to a use of the present composite material to fill cavities, preferably of a size smaller than 1 cm$^3$, of non-dental bone tissue, such as, for example, cavities produced by removed abscesses/cysts and/or tumours.

The invention will be illustrated here below, also with the aid of the attached figures, in which:

- Figure 1 shows an electron microscope (SEM) image of a fracture surface of the composite material of the invention which demonstrates the penetration of the polymer matrix into the interconnected through nanopores of the nanoporous APA microparticles of the invention;
- Figure 2 shows the results of elastic modulus measurements made with the Dynamic Mechanical Analysis (DMA) technique on the composite material of the invention and on a corresponding material containing non-porous micro-alumina;
- Figures 3a and 3b show the results of the artificial aging of a sample of the composite material of the invention and of two composite materials known in the art (Ceram-X® and Filtek™).

## Detailed description

In a first aspect, the present invention relates to the nanoporous anodic alumina in the form of microparticles, characterized in that it has interconnected through nanopores. Advantageously, the presence of said interconnected through nanopores enables the formation of a solid composite material, which is stable over time, by mechanical interlacing between the nanopores of the alumina and a polymer matrix, without the need for any coupling agent. Furthermore, the presence of the APA microparticles as a filler enables a totally biocompatible restorative composite material which is substantially not subject to a natural chemical degradation and endowed with excellent elasticity and resistance to aging.

In a preferred embodiment, the APA microparticles have a size of at least 5 micrometres ($\mu$m), preferably comprised between 5 and 20 micrometres, more preferably between 10 and 15 micrometres. Smaller sizes would result in a number of pores per microparticle that is not very convenient for use in the preparation of a composite material for dental use, whereas sizes larger than 20 micrometres could facilitate the formation of bacterial plaque following the use of said composite material as a dental material.

The pores of the APA of the invention can have a cylindrical shape or modulated shape, depending for example on the regime of the potential used during the anodization necessary for preparing the alumina. In any case, the interconnected through nanopores of the APA microparticles of the invention have an average diameter comprised between 20 and 300 nm, preferably between 80 and 250 nm, more preferably between 100 and 200 nm.

In a particularly preferred embodiment, the microparticles have a size of about 10 micrometres, and possess interconnected through nanopores having an average diameter of about 200 nanometres.

It has been noted, moreover, that the size and shape of the nanopores of the APA microparticles of the invention are particularly suitable for the functionalization thereof with various biologically active agents.

Therefore, in a further embodiment, the present invention relates to the nanoporous alumina in the form of microparticles, characterized in that it has interconnected through nanopores, and is functionalized with at least one biologically active agent. For this purpose, preferred biologically active agents are an antibacterial, disinfectant, mineralizing and/or regenerating agent, and are preferably selected from among: nanoparticles of silver, phosphate, fluoride, calcium or magnesium ions, proteins of the families of polylysine and extracellular matrix, integrin and laminin, vitronectin and fibronectin, bone morphogenetic proteins (BMP) and growth factors, preferably selected from among transforming growth factors (TGF), platelet-derived growth factors (PDGF) and insulin-like growth factors (IGF).

The functionalized alumina can be prepared by *in-situ* functionalization during the anodization step by suitably modifying the composition of the electrolytic solution used for the anodization and/or the anodization parameters, as described further below in the patent application. Alternatively, the functionalization can be *ex situ*, i.e. performed after the anodization and pore opening and before the formation of the microparticles by grinding. In an additional embodiment, the functionalized micro-nanoporous alumina of the invention can be obtained by both *in-situ* and *ex-situ* functionalization. In this manner it is possible to carry out a first functionalization (*in situ*), followed by a second functionalization (*ex situ*), for example through the addition of biologically active agents, not introducible with the first functionalization because of the anodization environment, which is hostile to the presence of biomolecules. Preferably, the nanoporous micro-alumina of the invention is in the form of microparticles having a size of 10 micrometres, has an average pore diameter of at least 50 nm and is preferably functionalized with antibacterial silver nanoparticles (with a diameter of between 5 and 50 nm), or with ions such as phosphate, fluoride, calcium and magnesium ions. In an additional aspect, the invention relates to a process for forming the nanoporous alumina in the form of microparticles described above, which comprises the steps of:

a) preparing a layer of APA by anodic oxidation (anodization) of an aluminium, preferably super-pure, electrode, immersed in an electrolytic solution;
b) forming a membrane of APA having interconnected through nanopores, by removing the residual aluminium substrate and subsequently by removing the bottom of the pores of the porous anodic alumina layer, and
c) grinding the alumina membrane of step b), obtaining microparticles of APA having interconnected through nanopores.

The step of anodizing the super-pure aluminium represents the actual step of growing the layer (or membrane) of anodic alumina. In practical terms, this step is carried out by immersing the super-pure aluminium electrode, generally in the form of a foil with a thickness of approximately 100 micrometres, in an electrolytic solution which is subjected to an electrochemical electrodeposition process. In this manner a planar layer of APA is obtained on the surface of the aluminium foil, which is subsequently freed of the residual aluminium substrate and converted into a membrane of nanoporous APA with interconnected through pores, as described below.

"Super-pure aluminium" means aluminium with a purity of 99.999%. Generally, the super-pure aluminium in the form of foil undergoes an "electropolishing" treatment prior to the anodization step. For this purpose, a typical treatment is carried out in an acid alcoholic solution, for example a solution, 1:5 by volume, of a perchloric acid: ethanol mixture refrigerated at a temperature comprised between 5 °C and 15 °C. Following the step of applying a current between the aluminium and an inert counter electrode, made for example of platinum, the final surface of the aluminium reflects like a mirror, i.e. is smooth on a nanometric scale, and ready for the subsequent anodization step.

Anodization can take place using techniques known in the art, which envisage the contact of the aluminium with an electrolytic solution in an electrochemical cell, leading to the formation of an APA membrane on the surface of the aluminium (see, for example, A.P. Li et al. Journal of Vacuum Science & Technology A 17:1428 (1999)).

The values of the electric potential required for the anodization process can vary according, for example, to the type of electrolyte present in the solution in which the super-pure aluminium is immersed. If a constant potential is used, it is possible to obtain alumina having cylindrical nanopores, whereas in the event of a variable potential, non-cylindrical nanopores, i.e. with a modulated diameter, can be obtained. The formation of nanopores with a modulated diameter is advantageous above all in cases where the alumina microparticles of the invention are used in the preparation of a composite material with a polymer matrix, as described below.

In fact, pores with a modulated diameter make it possible to obtain a high stability of the material and an increased bonding force between the polymer matrix and the microparticles of alumina as a filler.

In the case of anodization with a constant electric potential, the latter can be maintained at values selected in the interval from 10 Volts (V) to 200 V, while in the case of the anodization with a variable potential, the latter can vary, for example, between 100 V and 160 V. The electrolytic solution preferably used in the anodization step is an acid aqueous solution, preferably at a concentration comprised between 0.2 M and 0.6 M, more preferably between 0.3 M and 0.5 M.

Examples of preferred solutions are: an aqueous solution of phosphoric acid ($H_3PO_4$), or aqueous so-

lutions of sulphuric acid ($H_2SO_4$) or oxalic acid ($H_2C_2O_4$), preferably at concentrations comprised between 0.3 M and 0.5 M. In a particularly preferred embodiment, the constant potential anodization takes place using an electrolytic solution of oxalic acid, whereas the variable potential anodization can take place in an aqueous solution of sulphuric or phosphoric acid. For example, the anodization can be conducted in the presence of an aqueous solution of $H_3PO_4$ (preferably at a concentration of between 0.35 M and 0.45 M), at a constant potential comprised between 130 V and 150 V. In another example, the anodization can be conducted in the presence of an aqueous solution of $H_2SO_4$ at a constant potential selected between the values of 10 V and 30 V, or in the presence of $H_2C_2O_4$ at a constant potential selected between the values of 20 V and 60 V.

If a variable potential within the above-mentioned intervals of values is used, it is possible to obtain, in the nanoporous alumina of the invention, pores and distances between said pores (understood as the centre-to-centre distance between adjacent pores) which may be suitably varied in a controlled manner.

Preferably, the nanopores of the APA microparticles of the invention have a size comprised between 80 nm and 250 nm, more preferably between 100 and 200 nm. Preferably, the centre-to-centre distances between adjacent pores can vary between 100 and 300 nm, distances between pores comprised between 150 and 250 nm being particularly preferred.

The anodization step a) of the process of the present invention can also take place under galvanostatic conditions, i.e. under conditions of control of the current delivered. Preferred surface current density values are at least 80 mA/cm$^2$, more preferably at least 150 mA/cm$^2$.

In one embodiment, the anodization step a) of the process of the invention is carried out twice, according to a "2-step" procedure. Said procedure comprises a first anodization of the super-pure aluminium, preferably in the form of foil, at a constant potential, as described above, followed by a second anodization, preferably at the same constant potential as the first or else a variable potential, of longer duration. Preferably, the first anodization takes place at a constant potential selected in the interval of values comprised between 80 V and 130 V, for example for a period comprised between 1 and 2 hours, whereas the second anodization takes place at the same constant potential as the first, for example for a period comprised between 3 and 5 hours.

In one embodiment of the invention, the first layer of APA that is formed as a membrane on the aluminium foil after the first anodization is removed, typically by chemical attack in a selective liquid bath. For example, an aqueous solution of phosphoric acid: chromic acid is used, preferably in ratio comprised between 2:1 and 4:1 by weight. The temperature of removal can be comprised between 40° C and 60° C. The duration of the removal is generally comprised between 30 minutes and 2 hours, preferably around 1 hour, according to the concentration of the solution and the temperature used. After the removal of the first layer of APA, the super-pure aluminium foil is subjected to the second anodization described above.

When the second anodization of the "2-step" procedure is conducted at a variable potential, it can be carried out, for example, by alternating periods of 10-20 minutes with an electric potential varying between two identified values (for example, 100 and 160 V). The application of a variable potential, as mentioned previously, makes it possible to conveniently obtain a modulated diameter of the alumina pores, which corresponds to obtaining substantially non-cylindrical pores.

Non-cylindrical pores serve to increase the effect of mechanical interlacement between the nanoporous alumina microparticles of the invention and the polymer matrix during the preparation of a composite material that is useful as a restorative agent in dental applications. The presence of pores with a modulated diameter increases, in fact, the breaking strength of the resulting composite material, ensuring a longer life and resistance over time.

After the anodization step, which permits the preparation of the APA layer on the aluminium, before the removal of the pore bottoms the process comprises a step of selective removal of the remaining aluminium on the electrode on which the APA was grown during anodization. The selective removal of the aluminium generally takes place by immersion in a bath of a saturated aqueous solution of copper chloride ($CuCl_2$), according to techniques known in the art.

The next step of opening the APA pore bottoms ("pore opening"), previously closed by hemispherical caps, is carried out by treating the APA layer isolated from the aluminium electrode with an acid solution, for example of phosphoric acid. The treatment time is variable and comprised between 30 minutes and 1 hour. The treatment temperature is room temperature (i.e. comprised between 20° C and 35° C). Following the "pore opening" step, one obtains APA in the form of a membrane endowed with the interconnected through nanopores which characterize the APA microparticles of the invention.

As mentioned above, in a possible embodiment the APA can be functionalized in various ways with biologically active agents. This functionalization can take place during the anodization step (i.e. *in situ*, by introducing the biologically active agent directly into the electrolytic solution) and/or after the anodization step (i.e. *ex situ*) and before grinding.

Examples of possible functionalizations are with a biologically active agent, preferably an antibacterial, disinfectant, mineralizing and/or regenerating agent, selected from among: nanoparticles of silver, phosphate ions, fluoride ions, calcium ions or magnesium ions, or also proteins, preferably of the families of polylysine and extracellular matrix, integrin and laminin, vitronectin and fibronectin, bone morphogenetic proteins (BMP) and growth factors, preferably selected from among transforming growth factors (TGF), platelet-derived growth factors (PDGF) and insulin-like growth factors (IGF).

The in-situ functionalization can be carried out by adding the biologically active agent to the electrolytic solution and/or modifying the anodization conditions. The incorporation of said agent into the APA undergoing formation takes place during the step of applying electrical current during the constant potential and/or variable potential anodization process, as previously described, since under the above-specified conditions it is possible to observe the incorporation of an amount comprised between 3% and 8% by weight of anions of the respective salt in proportion to the amount of metal ions of aluminium. Should it be desired to increase the amount of incorporated ions it is possible to carry out the anodization under galvanostatic conditions as previously described.

In a preferred embodiment, the in-situ functionalization takes place during the galvanostatic anodization, using surface current density values of at least 80 $mA/cm^2$, preferably greater than 100 $mA/cm^2$, more preferably greater than 150 $mA/cm^2$. In this manner, the percentage of functionalization (understand as the amount by weight of a biologically active agent introduced into the pores relative to the weight of the aluminium ions included in the alumina of the APA) is increased compared to a corresponding in-situ functionalization performed via potentiostatic and/or variable potential anodization.

As an alternative or in addition to in-situ functionalization of the APA during the anodization step as described above, it is possible to carry out an *ex-situ* functionalization. Said *ex-situ* functionalization preferably takes place after the anodization steps, with the formation of the interconnected through nanopores, removal of the aluminium, and pore opening, and before the grinding step. The choice of the type of functionalization (*in-situ* or *ex-situ*) can be made, for example, according to the type of biologically active agent it is desired to introduce into the pores of microparticulate alumina. In fact, for example, whole silver nanoparticles, typically containing from thousands to hundreds of thousands of electrically neutral atoms, are difficult to incorporate by in-situ functionalization, i.e. during the anodization step, via the step of applying electric current. Therefore, the incorporation of silver nanoparticles must be carried out via an *ex-situ* functionalization. Analogously, functionalization with biomolecules, such as proteins and growth factors, as previously described, must be carried out *ex situ*; otherwise, when introduced into the acidic environment of the electrochemical anodization cell, and subjected to the electrical stresses of potential and current applied in said cell, they could easily become denatured.

Preferably, the step of *ex-situ* functionalization comprises the steps of:

- putting the APA having interconnected through nanopores in contact with a solution containing the selected biologically active agent;
- drying the functionalized APA; and
- subjecting the functionalized APA to calcination if necessary.

In particular, the APA membrane having interconnected through nanopores is put in contact, preferably by immersion, with an aqueous solution containing the biologically active agent with which it is intended to functionalize the APA. Said agent can be selected as specified above and in this case the respective aqueous solution will have a molarity comprised between 0.1 M and 3 M, preferably comprised between 0.3 M and 1 M. In general, it has been noted that concentrations lower than 0.1 M result in a scant incorporation of the bioactive agent, whereas concentrations higher than 3 M lead to an aggregation of molecules at the mouth of the pores and little penetration inside them, limited to depths inside the APA of less than one micrometre.

In the case of the above-mentioned biologically active agents, introduction into the pores can preferably take place without further chemical reactions, which could degrade the original functional characteristics of the molecules; for the same reason, with these bioactive agents it will be possible to avoid the calcination step, which would cause their degradation due to the thermal effects. Otherwise, inorganic materials can be introduced *ex situ* by means of a chemical reaction which may be necessary for the formation of the bioactive agent, and subsequent calcination to remove any undesirable reaction products. In a further preferred embodiment, the APA membrane is immersed in an ultrasound bath containing a solution, preferably aqueous, of the biologically active agent concerned. Using ultrasound advantageously enables better penetration of the solution of the biologically active agent into the pores, thus ensuring a better functionalization of the APA in terms of the amount of biologically active agent introduced. The contact between the nanoporous alumina and the solution is prolonged for a period of time that is generally comprised between 30 minutes and 2 hours, depending, for example, on the amount of material to be functionalized and/or the concentration of the aqueous solution or type of agent selected.

At the end of the step of immersion in an ultrasound bath, the functionalized alumina undergoes a drying step, typically in an oven, at a temperature comprised between 20 °C and 120 °C, preferably comprised between about 25 °C and about 50 °C in the case of organic active materials (biologically active agents as listed above, preferably proteins and growth factors), whereas in the case of inorganic materials (for example nanoparticles of silver or phosphate, fluoride, calcium and magnesium ions) the temperature is higher, preferably between 80 °C and 120 °C. This drying step enables the material to be prepared for the final calcination step, where necessary, in the case of inorganic bioactive agents, which do not degrade with this treatment. Calcination serves to remove the volatile and organic residues initially present together with the inorganic biologically active agent, thus completing the functionalization process. The calcination preferably takes place at a temperature comprised between about 400° C and about 600° C, for a period of time comprised between about 2 and 5 hours. For example, if $AgNO_3$ is used as the functionalizing agent, calcination enables the volatile part of the silver salt to be removed, so that only the metal (silver) remains inside the nanopores, and leaves the structure of the APA thus obtained substantially unchanged.

In a preferred embodiment, both functionalizations (*in-situ* and *ex-situ*) can be performed, thereby making it possible to obtain the nanoporous alumina microparticles of the invention functionalized in various ways and with combined properties, for example antibacterial and mineralizing.

As regards the final grinding step, this serves to convert the APA with interconnected through nanopores, optionally functionalized, into fragments of a size on the order of one micrometre or ten micrometres. Advantageously, the grinding makes it possible to obtain a population of microparticles of varying dimensions, with non-negligible dispersions (in terms of standard deviation of the linear dimension), on the order of at least ±50%. Where the aim is to produce a 'hybrid', as well as nanoporous, composite for dental restoration, this can enable a better packing of the filler, so that higher filling percentages can be reached compared to a monodisperse population of filler particles, which would not favour interstitial packing.

Grinding can take place using, for example, a ball mill, in a container made of zirconia (i.e. zirconium oxide), generally filled with balls made of the same material (zirconium oxide), in the presence of a solvent such as, for example, isopropyl alcohol or the like. The latter acts as a colloidal grinding medium, serving to homogenize the process by reducing the formation of aggregates during grinding and absorbing a large part of the heat produced by the various impacts and associated friction that occur in the grinding environment. Normally, in the presence of APA functionalized with an inorganic bioactive agent, distilled water is preferably used so as to minimize the risk of denaturing the agent. Alternatively, in the presence of an inorganic bioactive agent it is possible to use a smaller aliphatic alcohol (i.e. one having from 1 to 4 carbon atoms), preferably isopropanol, which is an even more preferred medium in the case of colloidal grinding.

After grinding, one obtain the porous alumina microparticles according to the invention, having a size of at least 5 micrometres, preferably comprised between 5 and 20 micrometres, more preferably between 10 and 15 micrometres.

In a further aspect, the invention relates to a use of the nanoporous APA in the form of microparticles obtained with the process of the invention, for the preparation of a composite material that is useful in the field of dental restoration. Therefore, one aspect of the present invention relates to a composite material for tooth restructuring comprising at least:

- nanoporous APA microparticles having interconnected through nanopores, optionally functionalized, as described above in detail; and
- a polymer matrix.

Thanks to the presence of said interconnected through nanopores, the nature of the bond between the APA microparticles and the polymer matrix in the composite material of the present invention is of a physical nature, rather than a chemical nature as in the prior art. Therefore, the present composite material does not require the use of any coupling agent between the alumina microparticles and polymer matrix, thus avoiding the problems tied to a possible release or the degradation that often accompanies similar materials known in the art, which require the use of a coupling agent (typically silane) to join the inorganic component and the polymer matrix.

In a preferred embodiment, the APA microparticles having interconnected through pores are present in the composite material of the invention in percentages comprised between 10% and 80% by weight relative to the weight of the composite material, preferably comprised between 40% and 75%, even more preferably between 50% and 70% by weight.

The polymer matrix can be selected from among the resins commonly used in the field of dentistry, such as, for example, acrylic and epoxy resins and the like and/or mixtures thereof.

In particular, in one embodiment of the invention, the polymer matrix comprises the monomers Bis-GMA (bisphenol A diglycidyl methacrylate) and TEGDMA (tetraethyleneglycol dimethacrylate), either alone or in a mixture, preferably present in a ratio comprised between 60:40 and 80:20, even more preferably in a ratio of 70:30. In particular, the use of a polymer matrix comprising the co-monomers Bis-GMA and TEGDMA makes it possible to obtain a high loading of the nanoporous APA microparticles within the polymer matrix. In this manner it is possible to obtain a composite material which preferably contains at least 40%, preferably at least 50%, even more preferably up to 70% by weight of nanoporous anodic alumina, so as to thereby enhance the mechanical properties and stability of the composite material obtained.

In addition to said monomers, the polymer matrix can also contain additional components, such as at least a photoinitiator, and possibly stabilizers, on their own or also mixed together. Each of such additional components is contained in the composite material in an amount equal to 0.5% of the monomer. In particular, in a preferred embodiment of the invention, the photoinitiator is camphorquinone and the stabilizer is 2-dimethylaminoethyl methacrylate (DMAEMA), preferably present in a 1:1 ratio.

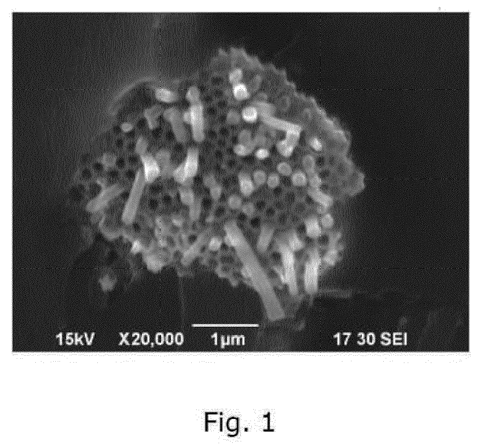

The composite material of the invention is illustrated in Fig. 1, where one can note the complete penetration of the polymer matrix into the interconnected through nanopores of the APA microparticles.

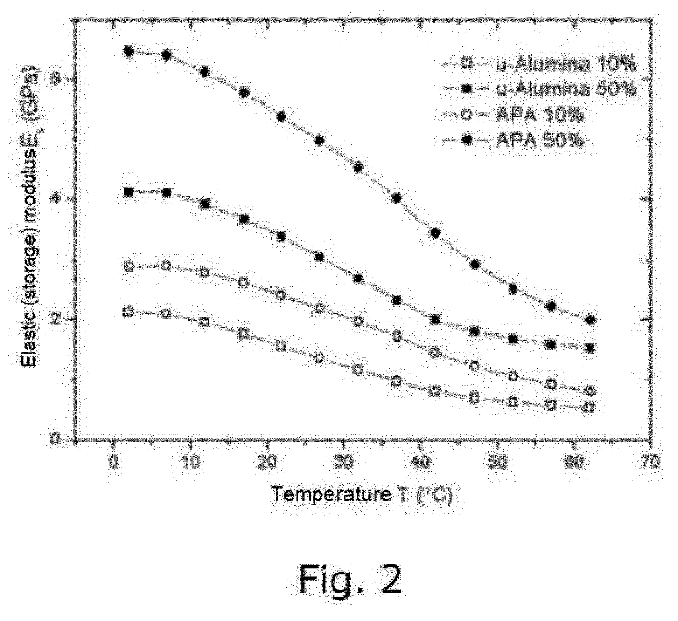

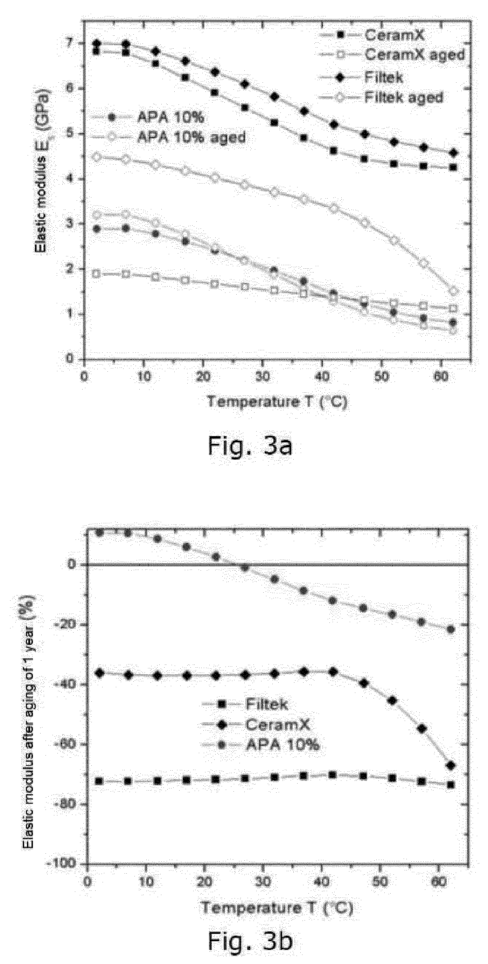

The comparative tests included in the present experimental part, moreover, demonstrate that the composite material of the invention has improved elasticity and improved stability over time, as indicated by the values of the elastic modulus (Fig. 2) and the comparative tests on aging ("artificial aging ") illustrated in Figs. 3a and 3b. In particular, Fig. 2 shows that the composite material of the invention has a higher elastic modulus than non-porous micro-alumina of similar dimensions with both 10% and 50% loading. Moreover, aging equivalent to one year was simulated for a bar of the composite material of the invention and of two different prior art composite materials based on a non-porous micro-filler, precisely, Ceram-X® and Filtek™ As can be seen from the graphs in Figs. 3a and 3b, after artificial aging the composite material of the invention shows a lower decrease in the elastic modulus than the two commercial compounds taken as reference. Moreover, at low temperatures, the aging to which the compound of the invention was subjected actually produces an effect of increasing the elastic modulus.

The process of preparing the composite material, which represents a further aspect of the invention, comprises the formation of the microparticulate APA as described above, which is then placed in contact with the selected polymer matrix. Therefore, in a further aspect, the invention relates to a process for preparing a composite material comprising at least APA microparticles and a polymer matrix, said process comprising the steps of:

- preparing nanoporous APA microparticles, optionally functionalized, as described above in detail and in accordance with each of the previous preferred embodiments; and
- mixing said microparticles with a polymer matrix.

The mixing with the polymer matrix preferably takes place by addition of the above-described APA microparticles, in the form of a granular powder, to the polymer matrix, under conditions of constant stirring and/or sonication.

The stirring and sonication conditions are selected in such a way as to permit the polymer matrix to penetrate effectively into the pores of the APA microparticles, which are optionally functionalized.

In a first step, the mixture of organic material (co-monomers, photoinitiator and stabilizer) is prepared by manual spatulation for at least 3 minutes. Subsequently, the microparticle filler is added under sonication, while manual spatulation is continuously performed for at least another 5 minutes. The composite mixture thus produced is poured into a glass mould (in which a layer about 1 mm thick is formed), and is then irradiated by means of a blue light LED (wavelength of 470 nm) with a power of 1 W, in 2 cycles of 20 seconds each.

In an additional aspect, the invention relates to the use of the composite material of the invention in the field of conservative dentistry, preferably as a material for dental restoration, for example as a filling for dental cavities, fissures or breaks.

In a further aspect, the invention relates to a method of tooth restructuring, preferably cosmetic, which comprises:

- positioning the composite material of the invention inside a dental cavity or fracture; and
- subjecting the material thus positioned to photopolymerization, preferably with one or more irradiation cycles.

As amply discussed, the present composite material can be advantageously used, for example, in the field of conservative dentistry, both as a cosmetic agent, for example in tooth reconstruction, and as a restorative material in the treatment of caries. Moreover, the present composite material shows a greater resistance to environmental chemical degradation than traditional compounds, thanks to the absence of silane coupling agents. The possibility of functionalizing the pores of the filler (i.e. of the microparticulate APA) with biologically active agents also increases the versatility and potential applications of the material, so that it can be applied both in the cosmetic field and for biomedical functions to treat surrounding tissues (antibacterial and mineralizing function). Thanks to the penetration of the resin into the pores of the rigid frame of the nanoporous alumina of the invention, finally, it is possible to obtain a composite material with greater breaking resistance, associated with a partial relaxation of the mechanical stress applied, both during polymerization of the resin and in working conditions under mastication. The present invention will now be described in detail in the experimental part below.

Experimental part

A sample of composite material comprising the nanoporous APA microparticles of the invention, not functionalized, and having a particle size of 5 micrometres and pore diameter of 200 nm, in a mixture with a polymer matrix comprising a co-monomer of bis-GMA and TEGDMA (in a ratio of 7:3 by weight), with the addition of camphorquinone and DMAEMA, each representing 0.5% by weight of the total mixture of co-monomers, underwent measurement of the mechanical properties using "Dynamic Mechanical Analysis" (DMA). A frequency of 1 Hz was considered, as this is similar to that of mastication, within a temperature interval compatible with that normally found in the oral cavity, into which both cold beverages and very hot foods (approximately between +5 °C and +60 °C) can be introduced. The same mechanical analysis was then performed on a sample of a composite material known in the art (Ceram-X®) and carried out with another experimental filler of equal composition and similar physical dimensions but not porous (micro-alumina). Finally, tests were also performed on a sample of composite material normally used in dental applications (Filtek™) containing a silane coupling agent. The results are illustrated in Fig. 2 and in Figs. 3a and 3b. In particular, in Fig. 2 it may be observed that at all of the temperatures considered, as regards both 10% and 50% loading, the composite material of the invention proves to have a higher elastic modulus than the material containing micro-alumina of similar dimensions, but not porous. Figs. 3a and 3b, on the other hand, show the results of simulated aging obtained by immersing a sample of the composite material of the invention in water heated to a temperature of up to about 60 °C, for a time set according to a model of artificial aging normally used on an industrial level (see for example C.G. Matasa, The Orthodontic Materials Insider 20. 1-4 (2008)). In detail, the formula adopted to compare times at different temperatures is the following:

$$ta/taa = Q_{10}^{(Taa-Ta)/10},$$

where:

taa    is the accelerated aging time elapsed at the temperature Taa;
ta      is the aging time that would have produced the same aging had the temperature remained at the operating level of real (not accelerated) aging Ta, lower, and
$Q_{10}$   is a coefficient ranging between 1.8 and 3, and in the present experiment is assumed to be equal to 2, which represents the relative increase in velocity characteristic of a generic chemical reaction when the temperature is increased by 10 °C.

An aging equivalent to 1 year was thus simulated under these conditions, both on a sample of the composite material of the invention and on two different samples of reference commercial materials, one of which selected because it is widely used and contains a silane coupling agent (Filtek™), and the other one because it is based on a ceramic micro-filler (similar, that is, to alumina) but is not porous (Ceram-X®). After aging, the DMA measurement was repeated and the pre- and post-artificial aging elastic modulus are shown in Fig. 3a. It is evident from the figure that both commercial materials (based on the filler-matrix bond of a chemical nature, mediated by the silane layer) see the elastic modulus decrease significantly at all temperatures considered, in a percentage of between 30% and 40% for Filtek™, and about 70% for Ceram-X®, whereas the composite material of the invention decreases by only 20% by weight at most and only at high temperatures. In conclusion, it has been demonstrated that after aging, the composite material of the invention comprising nanoporous alumina microparticles with interconnected pores and a polymer matrix shows a smaller decrease in the elastic modulus than the 2 reference commercial materials and at low temperatures the aging of the material of the invention even produces an increase in the elastic modulus.

## Claims

1.    An anodic porous alumina (APA) having interconnected through nanopores having diameter comprised between 20 and 300 nm, in the form of microparticles having a particle size of at least 5 micrometres.

2.    The anodic porous alumina according to claim 1, in the form of microparticles having a particle size comprised between 5 and 20 micrometres.

3.    The anodic porous alumina according to claim 1 or 2, functionalized with at least one biologically active agent.

4.    The anodic porous alumina according to claim 3, wherein said biologically active agent is an antibacterial, disinfectant, mineralizing and/or regenerating agent.

5.    The anodic porous alumina according to claim 4, wherein said biologically active agent is selected from: nanoparticles of silver, phosphate, fluoride, calcium or magnesium ions, proteins of the families of polylysine and extracellular matrix, integrin and laminin, vitronectin and fibronectin, bone morphogenetic proteins (BMP) and growth factors.

6.    A process for the formation of anodic porous alumina (APA) in the form of microparticles according to claim 1 or 2, which comprises the steps of:

a) preparing a layer of APA by anodic oxidation of an aluminium, preferably super-pure, electrode, immersed in an electrolytic solution;
b) forming a membrane of APA having interconnected through nanopores, by removing the residual aluminium substrate and subsequently by removing the bottom of the pores of the porous anodic alumina layer, and
c) grinding the alumina membrane of step b), obtaining APA microparticles having interconnected through nanopores.

7.    The process according to claim 6, further comprising a step of in-situ functionalization of the anodic porous alumina with a biologically active agent, conducted during step a), and/or an ex-situ functionalization step of the porous anodic alumina with a biologically active agent, preferably performed between step b) and step c).

8. The process according to claim 6 or 7, wherein step a) is carried out at a constant and/or variable electric potential, or under galvanostatic conditions.

9. A use of the anodic porous alumina having interconnected through nanopores in the form of microparticles according to any of claims 1 to 5, for the preparation of a composite material by mixing with a polymeric matrix.

10. A composite material comprising anodic porous alumina having interconnected through nanopores in the form of microparticles according to any one of claims 1 to 5, and a polymeric matrix.

11. The composite material according to claim 10, wherein said polymer matrix comprises the monomers bisphenol-A diglycidyl methacrylate and tetraethylenglycol dimethacrylate, alone or in a mixture.

12. The composite material according to claim 10 or 11 for use as a medicament.

13. The composite material for the use according to claim 12, as a filling material in dental recondition.

14. A method for dental restoration, preferably cosmetic, which comprises applying the composite material according to claim 10 or 11 in the tooth to be restored, and subsequently polymerizing said composite material applied.

**Patentansprüche**

1. Anodisches poröses Aluminiumdioxid (APA) mit durchgehend verbundenen Nanoporen mit einem Durchmesser zwischen 20 und 300 nm, in Form von Kleinstteilchen mit einer Teilchengröße von mindestens 5 Mikrometern.

2. Anodisches poröses Aluminiumdioxid nach Anspruch 1, in Form von Kleinstteilchen mit einer Teilchengröße zwischen 5 und 20 Micrometern.

3. Anodisches poröses Aluminiumdioxid nach Anspruch 1 oder 2, funktionalisiert mit mindestens einem biologisch aktiven Wirkstoff.

4. Anodisches poröses Aluminiumdioxid nach Anspruch 3, wobei genannter biologisch aktiver Wirkstoff ein antibakterieller, desinfizierender, mineralisierender bzw. regenerierender Wirkstoff ist.

5. Anodisches poröses Aluminiumdioxid nach Anspruch 4, wobei genannter biologisch aktiver Wirkstoff erlesen ist aus: Kleinstteilchen von Silber, Phosphaten, Fluoriden, Kalzium- oder Magnesiumionen, Proteinen aus den Familien von Polylysin und extrazellulärer Matrix, Integrin und Laminin, Vitronektin und Fibronektin, knochenbildungsanregenden Eiweißverbindungen (BMP) und Wachstumsfaktoren.

6. Verfahren zur Bildung des anodischen porösen Aluminiumdioxids (APA) in Form von Kleinstteilchen nach Anspruch 1 oder 2, die folgenden Schritte umfassend:

   a) Vorbereiten einer APA-Schicht durch anodische Oxidation eines Aluminiums, vorzugsweise super-rein, Elektrode, eingetaucht in einer Elektrolytlösung;
   b) Bilden einer APA-Membrane mit durchgehend verbundenen Nanoporen, durch Entfernen des verbleibenden Aluminiumsubstrats und anschließend durch Entfernen des Bodens der Poren der porösen anodischen Aluminiumdioxid-Schicht, und
   c) Schleifen der Aluminiummembrane von Schritt b), APA-Kleinstteilchen mit durchgehend verbundenen Nanoporen erhaltend.

7. Verfahren nach Anspruch 6, des Weiteren umfassend einen Schritt der vor Ort Funktionalisierung des anodischen porösen Aluminiumdioxids mit einem biologisch aktiven Wirkstoff, ausgeführt während Schritt a), und/oder ein ex-Situ Funktionalisierungsschritt des porösen anodischen Aluminiumdioxids mit biologisch aktivem Wirkstoff, vorzugsweise ausgeführt zwischen Schritt b) und Schritt c).

8. Verfahren nach Anspruch 6 oder 7, wobei Schritt a) ausgeführt wird bei konstantem und/oder variablem elektrischem Potential, oder unter galvanostatischen Bedingungen.

9. Verwendung des anodischen porösen Aluminiumdioxids mit durchgehend verbundenen Nanoporen in Form von Kleinstteilchen nach einem der Ansprüche von 1 bis 5, zur Vorbereitung eines Kompositmaterials durch Mischung mit einer Polymermatrix.

10. Kompositmaterial umfassend anodisches poröses Aluminiumdioxid mit durchgehend verbundenen Nanoporen in Form von Kleinstteilchen nach einem der Ansprüche von 1 bis 5, und eine Polymermatrix.

11. Kompositmaterial entsprechend Anspruch 10, wobei genannte Polymermatrix die Monomere Bisphenol-A Diglycidylmethacrylat und Tetraethylenglycoldimethacrylat umfasst, allein oder in Mischung.

**12.** Kompositmaterial nach Anspruch 10 oder 11 für die Verwendung als Medikament.

**13.** Kompositmaterial für die Verwendung nach Anspruch 12, als Füllmaterial in Zahnrestaurationen.

**14.** Verfahren für Zahnrestaurationen, vorzugsweise kosmetisch, umfassend das Einbringen des Kompositmaterials nach Anspruch 10 oder 11 in den zu restaurierenden Zahn, und anschließend das Polymerisieren des eingebrachten Kompositmaterials.

**Revendications**

**1.** Alumine poreuse anodique (APA) ayant des nanopores passants interconnectés ayant un diamètre compris entre 20 et 300 nm, sous forme de microparticules ayant une dimension de particule d'au moins 5 micromètres.

**2.** Alumine poreuse anodique selon la revendication 1, sous forme de microparticules ayant une dimension de particule comprise entre 5 et 20 micromètres.

**3.** Alumine poreuse anodique selon les revendications 1 ou 2, fonctionnalisée avec au moins un agent biologiquement actif.

**4.** Alumine poreuse anodique selon la revendication 3, dans laquelle ledit agent biologiquement actif est un agent antibactérien, désinfectant, minéralisant et/ou régénérant.

**5.** Alumine poreuse anodique selon la revendication 4, dans laquelle ledit agent biologiquement actif est sélectionné parmi : des nanoparticules d'argent, de phosphate, de fluorure, d'ions calcium ou magnésium, de protéines des familles de polylysine et de matrice extracellulaire, d'intégrine et de laminine, de vitronectine et de fibronectine, de protéines morphogénétiques osseuses (PMO) et de facteurs de croissance.

**6.** Procédé de formation d'alumine poreuse anodique (APA) sous forme de microparticules selon la revendication 1 ou 2, comprenant les étapes de :

    a) préparer une couche d'APA par oxydation anodique d'une électrode d'aluminium, de préférence super-pure, plongée dans une solution électrolytique ;
    b) former une membrane d'APA ayant des nanopores passants interconnectés en retirant le substrat d'aluminium résiduel et subséquemment en retirant le fond des pores de la couche d'alumine poreuse anodique, et
    c) moudre la membrane d'alumine de l'étape b), en obtenant des microparticules APA ayant des nanopores passants interconnectés.

**7.** Procédé selon la revendication 6, comprenant de plus une étape de fonctionnalisation in-situ de l'alumine poreuse anodique avec un agent biologiquement actif, conduite lors de l'étape a), et/ou une étape de fonctionnalisation ex-situ de l'alumine poreuse anodique avec un agent biologiquement actif, de préférence effectuée entre l'étape b) et l'étape c).

**8.** Procédé selon les revendications 6 ou 7, dans lequel l'étape a) est effectuée à un potentiel électrique constant et/ou variable, ou dans des conditions galvanostatiques.

**9.** Utilisation de l'alumine poreuse anodique ayant des nanopores passants interconnectés sous forme de microparticules selon l'une quelconque des revendications de 1 à 5, pour la préparation d'un matériau composite par mélange avec une matrice polymère.

**10.** Matériau composite comprenant de l'alumine poreuse anodique ayant des nanopores passants interconnectés sous forme de microparticules selon l'une quelconque des revendications de 1 à 5, et une matrice polymère.

**11.** Matériau composite selon la revendication 10, dans lequel ladite matrice polymère comprend les monomères bisphénol-A méthacrylate diglycidyl et dimethacrylate de tétraéthylèneglycol, seuls ou dans un mélange.

**12.** Matériau composite selon les revendications 10 ou 11 pour son utilisation en tant que médicament.

**13.** Matériau composite pour une utilisation selon la revendication 12, en tant que matériau de remplissage pour le reconditionnement dentaire.

**14.** Procédé de restauration dentaire, de préférence cosmétique, comprenant l'application du matériau composite selon les revendications 10 ou 11 dans la dent à restaurer et subséquemment la polymérisation dudit matériau composite appliqué.

Fig. 1

Fig. 2

Fig. 3a

Fig. 3b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0803241 A

- US 2011203928 A1

**Non-patent literature cited in the description**

- **A.P. LI et al.** *Journal of Vacuum Science & Technology A,* 1999, vol. 17, 1428
- **M. SALERNO et al.** *Bioinformation,* 2011, vol. 5 (7), 291-293

- **S. PRAVEEN et al.** *Dental Materials,* 2009, vol. 25, 296-301
- **C.G. MATASA.** *The Orthodontic Materials Insider 20,* 2008